# EUROPEAN PATENT SPECIFICATION

(11) **EP 3 969 618 B1**
(45) Date of publication and mention of the grant of the patent: **13.09.2023**
(21) Application number: 21712731.5
(22) Date of filing: 09.03.2021
(51) Int. Cl.: C12Q 1/6869

(54) **METHODS FOR SEQUENCING POLYNUCLEOTIDES**
VERFAHREN ZUR SEQUENZIERUNG VON POLYNUKLEOTIDEN
PROCÉDÉS DE SÉQUENÇAGE DE POLYNUCLÉOTIDES

(30) Priority: 09.03.2020 US 202062987035 P
(43) Date of publication of application: 23.03.2022
(73) Proprietor: Illumina, Inc., San Diego, CA 92122 (US)
(72) Inventor: PEACE, Jared, San Diego, California 92122 (US); BOJANOVIC MACHADO, Klara, San Diego, California 92122 (US); MCINERNEY, Peter, San Diego, California 92122 (US); BOUTELL, Jonathan, Mark, Cambridge Limited 19 Granta Park, Great Abington Cambridge CB21 6DF (GB)
(74) Representative: Gordon, Kirsteen Helen
(86) International application number: PCT/EP2021/055939
(87) International publication number: WO 2021/180733

(56) References cited:
- EP-A1- 2 470 669
- EP-A1- 3 155 122
- WO-A2-2008/041002
- US-A1- 2014 329 698

## Description

### FIELD

The present invention relates to improvements in methods of high throughput nucleic acid sequencing, and in particular to improvements to methods of carrying out extension reactions during pairwise sequencing.

### BACKGROUND

Nucleic acid sequencing methods have been known in the art for many years. Some such methods are based on successive cycles of incorporation of fluorescently labelled nucleic acid analogues. In such "sequencing by synthesis" or "cycle sequencing" methods the identity of the added base is determined after each nucleotide addition by detecting the fluorescent label.

In particular, US 5,302,509 describes a method for sequencing a polynucleotide template which involves performing multiple extension reactions using a DNA polymerase or DNA ligase to successively incorporate labelled polynucleotides complementary to a template strand. In such a "sequencing by synthesis" reaction, a new polynucleotide strand based-paired to the template strand is built up in the 5' to 3' direction by successive incorporation of individual nucleotides complementary to the template strand. The substrate nucleoside triphosphates used in the sequencing reaction are labelled at the 3' position with different 3' labels, permitting determination of the identity of the incorporated nucleotide as successive nucleotides are added.

In order to carry out accurate sequencing, a reversible chain-terminating structural modification or "blocking group" may be added to the substrate nucleotides to ensure that nucleotides are incorporated one at a time in a controlled manner. As each single nucleotide is incorporated, the blocking group prevents any further nucleotide incorporation into the polynucleotide chain. Once the identity of the last-incorporated labelled nucleotide has been determined, the label moiety and blocking group are removed, allowing the next blocked, labelled, nucleotide to be incorporated in a subsequent round of sequencing.

The technique of "paired-end" or "pairwise" sequencing (such terms are used interchangeably herein) is generally known in the art of molecular biology, particularly in the context of whole-genomic shotgun sequencing. Paired-end sequencing allows the determination of two "reads" of sequence from two places on a single polynucleotide duplex. The advantage of the paired-end approach is that there is significantly more information to be gained from sequencing two stretches each of "n" bases from a single template than from sequencing "n" bases from each of two independent templates in a random fashion. With the use of appropriate software tools for the assembly of sequence information it is possible to make use of the knowledge that the "paired-end" sequences are not completely random, but are known to occur on a single duplex, and are therefore linked or paired in the genome. This information has been shown to greatly aid the assembly of whole genome sequences into a consensus sequence.

Paired-end sequencing has typically been performed by making use of specialized circular shotgun cloning vectors. After cutting the vector at a specific single site, the template DNA to be sequenced (typically genomic DNA) is inserted into the vector and the ends resealed to form a new construct. The vector sequences flanking the insert DNA include binding sites for sequencing primers which permit sequencing of the insert DNA on opposite strands. However, the need for sequencing primers at both ends of the template fragment makes the use of array-based sequencing techniques extremely difficult. With array-based techniques, which usually rely on a single stranded template, it is generally only possible to sequence from one end of a nucleotide template, as the complementary strand is not attached to the surface.

In order to maximise the throughput of nucleic acid sequencing reactions, it is advantageous to be able to sequence multiple template molecules in parallel. Parallel processing of multiple templates can be achieved with the use of nucleic acid array technology. These arrays typically consist of a high-density matrix of polynucleotides immobilised onto a solid support material, and, generally rely on a single stranded template.

Various methods for double-ended sequencing of a polynucleotide template which can be carried out on a solid support have been reported. For example, methods of nucleic acid amplification, which allow amplification products to be immobilised on a solid support in order to form arrays comprised of clusters or "colonies" formed from a plurality of identical immobilised polynucleotide strands and a plurality of identical immobilised complementary strands are known. The nucleic acid molecules present in DNA colonies on the clustered arrays prepared according to these methods can provide templates for sequencing reactions.

In WO 2008/041002, a pairwise sequencing method is described comprising: (a) providing a solid support having immobilised thereon a plurality of double stranded template polynucleotides each formed from complementary first and second template strands linked to the solid support at their 5' ends, and multiple copies of one or more 5'- end immobilised primers capable of hybridising to the 3' end of the first template strand; (b) treating the plurality of double stranded template polynucleotides such that the first template strands are hybridised to 5' -end immobilised primers; (c) carrying out a first sequencing read to determine the sequence of a first region of the template polynucleotide; (d) carrying out an extension reaction to extend one or more of the immobilised primers to copy the first template strand to generate a second immobilised template strand; (e) treating the plurality of first and second immobilised template strands to remove the first template strand from the solid support; and (f) carrying out a second sequencing read to determine the sequence of a second region of the template polynucleotide, wherein determining the sequences of the first and second regions of the target polynucleotide achieves pairwise sequencing of said first and second regions of said target double-stranded polynucleotide.

US2014/329698 relates to methods for indexing samples and sequencing multiple polynucleotides templates.

There remains an ongoing need to improve sequencing platforms, including increasing run speed, improving efficiency, increasing accuracy and/or simplifying processes.

### SUMMARY

According to an aspect of the present invention there is provided a method for carrying out a strand resynthesis extension reaction during pairwise sequencing, wherein said strand resynthesis extension reaction is carried out between a first sequencing read and a second sequencing read, and wherein said strand resynthesis extension reaction extends one or more immobilised primers to copy a first template strand to generate a second immobilised template strand; characterised in that the strand resynthesis extension reaction is carried out using a non-thermostable strand displacement polymerase at a temperature of less than 55 °C, wherein said first template strands are a cluster, and wherein the same non-thermostable strand displacement polymerase is used during initial cluster generation and the strand resynthesis extension reaction.

According to an aspect of the present invention there is provided a method for carrying out an extension reaction during pairwise sequencing of first and second regions of a target polynucleotide, wherein said first and second regions are in the same target polynucleotide, said pairwise sequencing including the following steps:
(a) providing a solid support having immobilised thereon a plurality of first and second double stranded template polynucleotides each formed from a first template strand and its complement or a second template strand and its complement, wherein the first and second template strands are linked to the solid support at the 5' end and wherein the first or second template strand linked to the solid support further comprise a 5' immobilised extension primer;
(b) selectively removing the first and second template strand complements and selectively removing the second template strands to allow hybridisation of a first sequencing primer to the first template strand;
(c) carrying out a first sequencing read to determine the sequence of a first region of the template polynucleotide by a sequencing-by-synthesis technique or by a sequencing-by ligation technique;
(d) carrying out an extension reaction to extend one or more of the immobilised primers to copy the first template strand to generate a second immobilised template strand;
(e) selectively removing the first template strands to allow hybridisation of a second sequencing primer to the second template strands generated in step (d);
(f) carrying out a second sequencing read to determine the sequence of a second region of the template polynucleotide by a sequencing by synthesis technique or by a sequencing-by ligation technique, wherein determining the sequences of the first and second regions of the target polynucleotide achieves pairwise sequencing of said first and second regions of said target polynucleotide;
wherein the step (d) extension reaction is carried out using a non-thermostable strand displacement polymerase at a temperature of less than 55°C, wherein said first template strands are a cluster, and wherein the same non-thermostable strand displacement polymerase is used during initial cluster generation and the strand resynthesis extension reaction.

It has surprisingly been found that the extension reaction carried out after the first sequence read can be carried out at lower temperatures with non-thermostable strand displacement polymerases and yet still retain high levels of strand resynthesis. Using non-thermostable strand displacement polymerases may also lead to further advantages, including: faster regeneration times; simplified protocols including reductions in the process steps; reduction in the number of different reagents used and/or amount of reagents used; reducing complexity of the chemistry; and/or reducing complexity of the cartridge used to carry out the methods.

According to a further aspect, there is provided a method for pairwise sequencing of first and second regions of a target polynucleotide, wherein said first and second regions are in the same target polynucleotide, said pairwise sequencing including the extension reaction as described herein.

According to a further aspect, there is provided a method of improving the data quality of a sequencing reaction, comprising carrying out an extension reaction as described herein.

### BRIEF DESCRIPTION OF THE DRAWINGS

FIGs. 1 to 4 show exemplary extension reaction workflows according to the present invention.
FIG. 5 shows the regeneration efficiency of protocols according to the present invention.
FIGs. 6A and 6B show optimisation analysis for protocols according to the present invention.
FIG.7 shows a schematic of bridge amplification for paired-end resynthesis.

### DETAILED DESCRIPTION

The following features apply to all aspects of the invention.

Sequencing generally comprises four core steps: 1) library preparation to form a plurality of template polynucleotides available for sequencing; 2) cluster generation to form an array of amplified single template polynucleotides on a solid support; 3) sequencing the cluster array; and 4) data analysis to determine the target sequence.

The present invention as defined in the appended claims is directed to the regeneration step (also referred to herein as the strand resynthesis extension reaction or cluster regeneration) that is carried out between the first and second sequencing steps during a pairwise sequencing process. The present invention is directed to the use of non-thermostable strand displacement polymerases during said extension reaction.

The typical steps of pairwise sequencing are known and have been described in WO 2008/041002. However, the key steps will be briefly described. Methodology applicable to the present invention have been described in WO 08/041002, WO 07/052006, WO 98/44151, WO 00/18957, WO 02/06456, WO 07/107710, WO05/068656, and US 2012/0316086,. Further information can be found in US 20060024681, US 200602926U, WO 06110855, WO 06135342, WO 03074734, WO07010252, WO 07091077, WO 00179553 and WO 98/44152,.

Suitable templates for sequencing can be prepared by solid-phase nucleic acid amplification to produce nucleic acid colonies. The templates to be amplified (and then sequenced) will generally comprise unknown regions flanked by known ends comprising universal primers, for example prepared according to methods described in application WO07052006,. For example, the templates may derive from a sample of genomic DNA, or from a cDNA library. The amplification can be done using procedures analogous to those described in WO 98/44151, WO 00/18957, WO0206456 or WO07107710,. If the template nucleic acid is formed by solid-phase nucleic acid amplification, these non-target sequences may be derived from the primers used for the amplification reaction. Alternatively, the non-target sequences may be ligated to fragmented target sequences to incorporate them into the nucleic acid molecule.

For example, suitable nucleic acids to be amplified with universal primers may be prepared by modifying polynucleotides comprising the target region to be amplified (and sequenced) by addition of known adaptor sequences to the 5' and 3' ends of the target polynucleotides to be amplified.

The adaptors are typically short oligonucleotides that may be synthesised by conventional means. The adaptors may be attached to the 5' and 3' ends of target nucleic acid fragments by a variety of means (e.g. subcloning, ligation. etc). More specifically, two different adaptor sequences are attached to a target nucleic acid molecule to be amplified such that one adaptor is attached at one end (i.e. the 5' or 3' end) of the target nucleic acid molecule and another adaptor is attached at the other end (i.e. the 3' or 5' end respectively) of the target nucleic acid molecule. The resultant construct comprising a target nucleic acid sequence flanked by adaptors may be referred to herein as a "substrate nucleic acid construct" or as a "template polynucleotide" or "template construct". The template polynucleotides may advantageously be size-fractionated prior to modification with the adaptor sequences.

The adaptor sequences contain sequences that permit amplification of these molecules on a solid support to form clusters using forward and reverse primers (e.g. S1 and S2) immobilised on the solid support (the general structure of these are described in further detail below). These sequences in the adaptors may be referred to herein as "primer binding sequences". In order to act as a template for nucleic acid amplification, a single strand of the template construct must contain a sequence which is complementary to the forward amplification primers (e.g. S1) such that the forward primer molecule can bind and prime synthesis of a complementary strand by copying the template and a sequence which is complementary to the reverse amplification primer (e.g. S2) such that again the reverse primer molecule can bind and prime synthesis of a second complementary strand. The sequences in the adaptors which permit hybridisation to the immobilised primer molecules will typically be around 20-40 nucleotides in length, although the invention is not limited to sequences of this length.

The precise identity of sequences S1 and S2 in the amplification primers, and hence the cognate sequences in the adaptors, are generally not material to the invention, as long as the primer molecules are able to interact with the adaptor sequences in order to direct PCR amplification. The criteria for design of PCR primers are generally well known to those of ordinary skill in the art.

The template polynucleotides may also be prepared to further contain a tag or index sequence. The use of a tag sequence, for example as described in application WO05068656, allows multiple different samples to be analysed in the same sequencing run whilst preserving the identity of each sample. The tag can be read at the end of the first read and/or at the start (or end) of the second read. The invention is not limited to two reads per cluster, three or more reads per cluster are obtainable simply by dehybridising a first extended sequencing primer, and rehybridising a second primer before or after the strand resynthesis extension reaction. Methods of preparing suitable samples for indexing are described in the prior art.

In one example, a template polynucleotide comprises in the 5' to 3' direction, a first primer-binding sequence (e.g. P5), an index sequence (e.g. i5), a first sequencing binding site (e.g. SBS3), an insert, a second sequencing binding site (e.g. SBS12'), a second index sequence (e.g. i7') and a second primer-binding sequence (e.g. P7'). In another embodiment, the template comprises, in the 3' to 5' direction, a first primer-binding site (e.g. P5', which is complementary to P5), an index sequence (e.g. i5', which is complementary to I5), a first sequencing binding site (e.g. SBS3' which is complementary to SBS3), an insert, a second sequencing binding site (e.g. SBS12, which is complementary to SBS12), a second index sequence (e.g. i7, which is complementary to I7) and a second primer-binding sequence (e.g. P7, which is complementary to P7'). Either template is referred to herein as a "template strand" or "a template polynucleotide". The combination of a primer-binding sequence, an index sequence and a sequencing binding site may be referred to herein as an adaptor sequence, and a single insert is flanked by a 5' adaptor sequence and a 3' adaptor sequence.

The sequence of the P5 primer-binding sequence may comprise SEQ ID NO: 1 or a variant thereof, the sequence of the P5' adaptor may comprise SEQ ID NO: 3 or a variant thereof, the sequence of the P7 adaptor may comprise SEQ ID NO: 2 or a variant thereof and the sequence of the P7' adaptor may comprise SEQ ID NO: 4 or a variant thereof. In embodiments, the variant has at least 80% sequence identity to SEQ ID NO: 1, 2, 3 or 4. More preferably, the variant has at least 86%, 87%, 88%, 89%, 90%, 91%, 92%, 93%, 94%, 95%, 96%, 97%, 98%, or at least 99% overall sequence identity to SEQ ID NO: 1, 2, 3 or 4.
SEQ ID NO: 1: P5 sequence
   AATGATACGGCGACCACCGAGATCTACAC
SEQ ID NO: 2: P7 sequence
   CAAGCAGAAGACGGCATACGAGAT
SEQ ID NO: 3 P5' sequence (complementary to P5)
   GTGTAGATCTCGGTGGTCGCCGTATCATT
SEQ ID NO: 4 P7' sequence (complementary to P7)
   ATCTCGTATGCCGTCTTCTGCTTG

The next core step following library preparation, is cluster generation to form an array of amplified single template polynucleotides on a solid support. As explained above, for amplification of the template polynucleotides to proceed, a mixture of at least two amplification primers is immobilised or "grafted" onto the surface of a suitable solid support.

As used herein, the term "solid support" refers to a rigid substrate that is insoluble in aqueous liquid (or at least sufficiently rigid that the substrate does not swell substantially when taking up the liquid and does not contract substantially when the liquid is removed by drying). The invention may make use of solid supports comprised of a substrate or matrix (e.g. glass slides, polymer beads etc.) which has been "functionalised", for example by application of a layer or coating of an intermediate material comprising reactive groups which permit covalent attachment to biomolecules, such as polynucleotides. The solid support may be a planar substrate. The solid support may be a laminar substrate. The solid support may be beads. Further examples of materials that can be used in the structured substrates or methods of the present disclosure are described in US Pat. App. Pub. No. 2012/0316086 A1,.

As a first step in colony generation by solid-phase amplification a mixture of forward and reverse amplification primers may be immobilised or "grafted" onto the surface of a suitable solid support.

When referring to immobilisation or attachment of molecules (e.g. nucleic acids) to a solid support, the terms "immobilised" and "attached" are used interchangeably herein and both terms are intended to encompass direct or indirect, covalent or non-covalent attachment, unless indicated otherwise, either explicitly or by context. In certain embodiments of the invention covalent attachment may be preferred, but generally all that is required is that the molecules (e.g. nucleic acids) remain immobilised or attached to the support under the conditions in which it is intended to use the support, for example in applications requiring nucleic acid amplification and/or sequencing. When referring to attachment of nucleic acids to other nucleic acids, then the terms "immobilised" and "hybridised" are used herein, and generally refer to hydrogen bonding between complementary nucleic acids.

In one example, the grafting step will generally involve covalent attachment of the primers to the support at or near the 5' end, leaving the 3' end free for primer extension. The amplification primers are typically oligonucleotide molecules having the following structures:
Forward primer: A-L-S1
Reverse primer: A-L-S2

Wherein A represents an optional moiety which allows attachment to a solid support, L represents an optional linker moiety and S1 and S2 are polynucleotide sequences which permit amplification of a substrate nucleic acid molecule comprising a target region that it is desired to (fully or partially) sequence. Sequences S1 and S2 in the amplification primers may be specific for a particular target nucleic acid that it is desired to amplify, but in other embodiments sequences S1 and S2 may be "universal" primer sequences which enable amplification of any target nucleic acid of known or unknown sequence which has been modified (e.g. with adaptors as described above) to enable amplification with the universal primers.

The mixture of primers grafted onto the solid support will generally comprise substantially equal amounts of the forward and reverse primers.

Group A can be any moiety (including a non-nucleotide chemical modification) which enables attachment (preferably covalent) to a solid support. Group A may comprise a sulphur-containing nucleophile, such as phosphorothioate, present at the 5' end of a polynucleotide strand. Alternatively, group A may be omitted where suitable chemistry is used to directly attach either the linker or the nucleic acid directly to the solid support.

L represents a linker or spacer which may be included but is not strictly necessary. The linker may be included in order to ensure that a cleavage site present in the immobilised polynucleotide molecules generated as a result of the amplification reaction is positioned at an optimum distance from the solid support, or the linker may itself contain a cleavage site.

The linker may be a carbon-containing chain with a formula (CH₂)ₙ wherein "n" is from 1 to about 1500, for example less than about 1000, preferably less than 100, e.g. from 2-50, particularly 5-25. However, a variety of other linkers may be employed with the only restriction placed on their structures being that the linkers are stable under conditions under which the polynucleotides are intended to be used subsequently, e.g. conditions used in DNA amplification and sequencing.

Linkers which do not consist of only carbon atoms may also be used. Such linkers may include polyethylene glycol (PEG)

Linkers formed primarily from chains of carbon atoms and from PEG may be modified so as to contain functional groups which interrupt the chains. Examples of such groups include ketones, esters, amines, amides, ethers, thioethers, sulfoxides, sulfones. Separately or in combination with the presence of such functional groups may be employed alkene, alkyne, aromatic or heteroaromatic moieties, or cyclic aliphatic moieties (e.g. cyclohexyl). Cyclohexyl or phenyl rings may, for example, be connected to a PEG or (CH₂)ₙ chain through their 1- and 4-positions.

As an alternative to the linkers described above, which are primarily based on linear chains of saturated carbon atoms, optionally interrupted with unsaturated carbon atoms or heteroatoms, other linkers may be envisaged which are based on nucleic acids or monosaccharide units (e.g. dextrose). It is also within the scope of this invention to utilise peptides as linkers.

In a further embodiment the linker may comprise one or more nucleotides. Such nucleotides may also be referred to herein as "spacer" nucleotides. Typically, from 1 to 20, more preferably from 1 to 15 or from 1 to 10, and more particularly 2, 3, 4, 5, 6, 7, 8, 9 or 10 spacer nucleotides may be included. Most preferably the primer will include 10 spacer nucleotides. It is preferred to use polyT spacers, although other nucleotides and combinations thereof can be used. In one preferred embodiment the primer may include 10T spacer nucleotides.

For the primer grafting reaction to proceed a mixture of the amplification primers is applied to a solid support under conditions which permit reaction between moiety A (if present) and the support, or between the nucleic acid and the support. The solid support may be suitably functionalised to permit covalent attachment via moiety A. The result of the grafting reaction is a substantially even distribution of the primers over at least a portion of the solid support. Where the solid support includes nanowells, then in preferred embodiments primers are restricted to the location of the nanowells, and are not present in interstitial regions of the solid support.

Following attachment of the amplification primers, the solid support is contacted with the template polynucleotides to be amplified under conditions which permit hybridisation between the template and the immobilised primers. In the example given above, the P5' and P7' primer-binding sequences on the template polynucleotides are complementary to immobilised primers (P5 and P7 respectively) present on the surface of the solid support. As used herein "'" denotes the complementary strand.

The template is usually added in free solution under suitable hybridisation conditions, which will be apparent to the skilled reader. Typically, hybridisation conditions are, for example, 5xSSC at 40°C. Solid-phase amplification can then proceed (e.g. by the method analogous to that of WO 98/44151) the first step of the amplification being a primer extension step in which nucleotides are added to the 3' end of the immobilised primer hybridised to the template to produce a fully extended immobilised complementary strand (or polynucleotide duplex). This complementary strand will thus include at its 3' end a sequence which is capable of binding or "bridging" to a second primer molecule immobilised on the solid support - leading to a new round of amplification starting from extension of the second immobilised primer using the complementary strand as a template.

Subsequent amplification reactions may then proceed substantially as described in WO 98/44151 or that of WO 00/18957, which will result in the production of a clustered array comprised of colonies of "bridged" amplification products. Here both strands of the amplification products will be immobilised on the solid support at or near the 5' end, this attachment being derived from the original attachment of the amplification primers. Typically, the amplification products within each colony will be derived from amplification of a single template (target) molecule. Alternatively, when generating clusters, other amplification procedures may be used, and will be known to the skilled person. For example, amplification may be isothermal amplification using a strand displacement polymerase; or may be exclusion amplification as described in WO 2013/188582.

Modifications required to enable subsequent cleavage of the bridged amplification products may be advantageously included in one or both amplification primers. Such modifications may be placed anywhere in the amplification primer, provided this does not affect the efficiency of the amplification reaction to a material extent. Thus, the modifications which enable cleavage may form part of the linker region L or one or both of sequences S1 or S2. By way of example, the amplification primers may be modified to include inter alia diol linkages, uracil nucleotides, ribonucleotides, methylated nucleotides, peptide linkers, PCR stoppers or recognition sequences for a restriction endonuclease. Because all nucleic acid molecules prepared by solid-phase amplification will ultimately contain sequences derived from the amplification primers, any modifications in the primers will be carried over into the amplified products.

In this context, the term "solid-phase amplification" refers to an amplification reaction which is analogous to standard PCR, except that the forward and/or reverse amplification primers are immobilised (e.g. covalently attached) to a solid support at or near the 5' end. The products of the PCR reaction are thus extended strands derived by extension of the amplification primers that are immobilised on the solid support at or near the 5' end. Solid-phase amplification may itself be carried out, for example, using procedures analogous to those described in WO 98/44151 and WO 00/18957.

A polynucleotide duplex will typically be formed from two complementary polynucleotide strands comprised of deoxyribonucleotides joined by phosphodiester bonds, but may additionally include one or more ribonucleotides and/or non-nucleotide chemical moieties and/or non-naturally occurring nucleotides and/or non-naturally occurring backbone linkages. In particular, the double-stranded nucleic acid may include non-nucleotide chemical moieties, e.g. linkers or spacers, at the 5' end of one or both strands. By way of non-limiting example, the double-stranded nucleic acid may include methylated nucleotides, uracil bases, phosphorothioate groups, ribonucleotides, diol linkages, disulphide linkages, peptides etc. Such non-DNA or non- natural modifications may be included in order to permit cleavage, or to confer some other desirable property, for example to enable covalent attachment to a solid support, or to act as spacers to position a site of cleavage an optimal distance from the solid support.

Where a polynucleotide strand is only partially hybridised to a complementary strand - for example, a long polynucleotide strand hybridised to a short nucleotide primer - it may still be referred to herein as a single stranded nucleic acid.

The next core step is sequencing the cluster array.

To facilitate sequencing, it is preferable if one of the strands is removed from the surface to allow efficient hybridisation of a sequencing primer to the remaining immobilised strand - e.g. the second strand is removed leaving the first strand remaining or vice versa. Suitable methods for linearisation are described below, and described in more detail in application number WO07010251,.

Denaturation (and subsequent re-annealing of the cleaved strands) results in the production of a sequencing template which is partially or substantially single-stranded. A sequencing reaction may then be initiated by hybridisation of a sequencing primer to the single-stranded portion of the template. In embodiments of the invention, sequencing is carried out using a strand-displacement polymerase enzyme as described below. The methods may require two sequencing reactions to be performed. The first sequencing reaction is initiated by a first sequencing primer added from solution and leads to sequencing of a first template strand or may be initiated by the 3' -hydroxyl group of the immobilised primer that is freed from within the immobilised duplex. The second sequencing reaction is initiated by a second sequencing primer that can either be immobilised or applied in solution. Hybridisation of the sequencing primer in solution to the (first) template strand is achieved by contacting the primer and template strand under conditions which promote annealing of primer to template. Such conditions will generally be well known to those skilled in the art of molecular biology. Nonetheless, this method allows sequence data to be obtained from both ends of a template polynucleotide by obtaining a sequence read from one strand of the template, copying the strand using immobilised primers (as explained below), releasing the first strand and sequencing the second, copied strand. This gives a sequence read from both ends of the original fragment.

As such, in one example the invention relates to methods for sequencing regions of a polynucleotide template, referred to herein as the first and second regions for sequence determination. The first and second regions for sequence determination are at both ends of a polynucleotide template, (the template and its complement are referred to herein respectively as first and second template strands). Once the sequence of a strand is known, the sequence of its complementary strand is also known, therefore the term two regions can apply equally to both ends of a single stranded template, or both ends of a double stranded template, wherein a first region and its complement are known, and a second region and its complement are known.

In one example, the first sequencing read may comprise the binding of a first sequencing primer (read 1 sequencing primer) to the first sequencing binding site (e.g. SBS3') followed by synthesis and sequencing of the complementary strand. This leads to the sequencing of the insert. In a second step, an index sequencing primer (e.g. i7 sequencing primer) binds to a second sequencing binding site (e.g. SBS12) leading to synthesis and sequencing of the index sequence (e.g. sequencing of the i7 primer). Following cluster regeneration (as described herein) is the second sequence read. The second sequencing read may comprise binding of an index sequencing primer (e.g. i5 sequencing primer) to the complement of the first sequencing binding site on the template (e.g. SBS3) and synthesis and sequencing of the index sequence (e.g. i5). In a second step, a second sequencing primer (read 2 sequencing primer) binds to the complement of the primer (e.g. i7 sequencing primer) binds to a second sequencing binding site (e.g. SBS12') leading to synthesis and sequencing of the insert in the reverse direction. In other words, SBS3 is the sequencing primer for Read1, SBS12 for Read2, SBS3' for i5, SBS12' for i7.

Sequencing can be carried out using any suitable "sequencing-by-synthesis" technique, wherein nucleotides or oligonucleotides are added successively to a free 3' hydroxyl group, typically provided by annealing of a sequencing primer, resulting in synthesis of a polynucleotide chain in the 5' to 3' direction. In a particular embodiment, the nature of the nucleotide or oligonucleotide added is determined after each addition.

One particular sequencing method relies on the use of modified nucleotides that can act as reversible chain terminators. Suitable nucleotides are described in WO04018497. Once the modified nucleotide has been incorporated into the growing polynucleotide chain complementary to the region of the template being sequenced there is no free 3'-0H group available to direct further sequence extension and therefore the polymerase can not add further nucleotides. Once the nature of the base incorporated into the growing chain has been determined, the 3' block may be removed to allow addition of the next successive nucleotide. By ordering the products derived using these modified nucleotides it is possible to deduce the DNA sequence of the DNA template. Such reactions can be done in a single experiment if each of the modified nucleotides has attached thereto a different label, known to correspond to the particular base, which facilitates discrimination between the bases added at each incorporation step. Suitable labels are described in WO2007/135368. Alternatively, a separate reaction may be carried out containing each of the modified nucleotides, which are added separately. The modified nucleotides may also carry a label to facilitate their detection. In a particular embodiment, the label is a fluorescent label. Each nucleotide type may carry a different fluorescent label. However the detectable label need not be a fluorescent label. Any label can be used which allows the detection of the incorporation of the nucleotide into the DNA sequence.

One method for detecting the fluorescently labelled nucleotides comprises using laser light of a wavelength specific for the labelled nucleotides, or the use of other suitable sources of illumination. The fluorescence from the label on an incorporated nucleotide may be detected by a CCD camera or other suitable detection means. Suitable detection means are described in in the prior art.. Once the first sequencing read is complete, and sufficient read length has been determined, the rest of the strand can be copied. If the 3' -hydroxyl group was originally created with a nicking enzyme, then it will be possible to re-create a fresh 3'-hydroxyl group at the same position, and extend from this position, however it is equally possible to continue to copy the first template strand from the 3'-hydroxyl group of the nucleotides incorporated as part of the sequencing reaction. This extension reaction with all four unlabelled nucleotides and a polymerase will copy all the bases of first template. The immobilised primers may contain a restriction site for a nicking enzyme, and treatment with the restriction enzyme may shorten the immobilised primers, or the immobilised template duplexes to release an unblocked 3' hydroxyl group. Methods of generating a free 3'-hydroxyl in only one strand of a duplex include either treatment with a nicking enzyme, or chemical treatment to remove a specific nucleotide. Suitable nicking enzymes are well known in the art, and would preferably cut at a site that is 3' -remote to their binding site to avoid having to sequence bases that derive from the known nicked site. The nicking enzyme should cut only one of the strands, at the end closest to the surface. Examples of suitable restriction enzymes would include Nt.BstNBI and Nt.Alwl, which have no bases of defined sequence beyond the released 3' -hydroxyl.

After the first sequencing run, the nucleotides undergo a regeneration step (as discussed further herein) to enable the second sequencing run. Thus, the nucleotides can be deprotected to allow further cycles of copying the template strand. If the nucleotides carry a dideoxy modification, this can be removed using an exonuclease, or a polymerase with exonuclease activity. Thus, the clusters could be made with two grafted primers as described, the unused primers blocked during the first sequencing reaction, then deblocked to allow further copying of the template strands. A portion of the amplification primers may be attached to the surface with a modification blocking the 3' hydroxyl from extension in the amplification cycles. This in effect means that the surface is treated with three or more amplification primers rather than two. At least two of the amplification primers should comprise regions of identical sequence, but at least one primer will not be susceptible to the conditions used to remove the second primer during the linearisation process, and will contain a 3'- blocking moiety. The blocking moiety may take the form of a chemical block, such as an azidomethyl group that can be removed with a phosphine reagent, an enzymatically removable such as a phosphate group that can be removed with a phosphatase, or may be in the form of a nucleoside group that can be removed using 3' -5' exonucleolysis. Such nucleoside modifications include abasic sites, that can be removed as described, or 2', 3' dideoxy nucleotides that can be removed by a polymerase with exonuclease activity. Further modifications include using an oligonucleotide sequence that can form a self complementary region with a recognition sequence for a restriction enzyme. Treatment with the restriction enzyme should cut the hairpin strand and release a shorter sequence with a free 3' hydroxyl group. Treatment of the surface after the first sequencing run is completed to deblock the primers will allow the remaining first strand to hybridise to the deprotected primers and recopy the already sequenced strands.

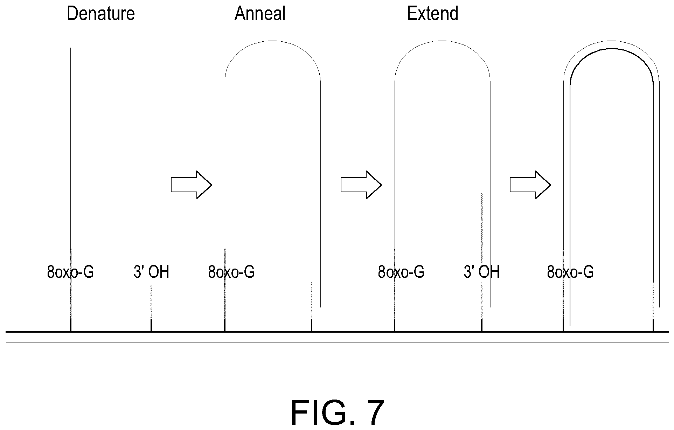

Once the sequencing product of the first sequencing read has been removed the first template strand will remain immobilised on the solid support. As the first template strand includes at its 3' end a sequence complementary to a second primer immobilised on the solid support it is capable of binding or "bridging" to that second primer molecule. An extension reaction can then be carried out as further described herein to extend one or more of the immobilised primers to copy the first template strand and generate a second immobilised template strand, which is subsequently sequenced in the second sequence read. Thus, the cluster can be regenerated to enable the second sequence read. This step may be repeated a number of times to obtain paired end resynthesis. Figure 7 shows one example of this cycle. Here bridge amplification requires the cycling of three reagents to perform rounds of multiplication. First a denaturation agent (LDR) is used to denature clusters at 55°C. H₂O is then pushed across the solid support (e.g. a flow cell) to facilitate removal of the denaturation agent. A strand-displacement polymerase enzyme is then used to extend the available 3' surface primers. This cycle may be repeated as described below for paired-end re-synthesis.

Again, this re-generates the template duplexes where both strands are immobilised. In relation to the extension reaction during pairwise sequencing (i.e. between the first and second sequencing reads), extension is carried out using a strand-displacement polymerase enzyme.

In a further step, it may be advantageous to extend the free 3'-hydroxyl primers with a plurality of bases complementary to the template prior to initiating sequencing. This both raises the melting temperature of the immobilised duplex, and helps prevent the template strand from re-hybridising to other immobilised primers during sequencing, which gives rises to phasing problems within the cluster. The ligation step is carried out after the phosphatase step has removed the phosphate group from the immobilised primer. Addition of 20-30 bases of sequence can be performed by a ligation reaction with a 5' - phosphate modified primer hybridised adjacent to the free 3' -hydroxyl. A ligase, such as T4 DNA ligase can be used to seal the gap. In the case of USER treatment which removes the U nucleotide, the 5' -base of the primer will be T that replaces the excised U. For the hybridisation step to be carried out efficiently, the 5'-non immobilised strand must have been removed by 5' -3' exonucleolysis treatment as described above. Such immobilised, extended primers with a free 3' -hydroxyl are described as extended 5' - anchored, or extended immobilised primers, and generation of such extended primers is only one of the steps involved in treating the plurality of template polynucleotides such that the first template strand is hybridised to a primer that is immobilised on the solid support at its 5 '-end.

In one example, prior to undertaking the extension reaction described above, it may be advantageous to extend the immobilised primer. The extension can be performed using a hybridised oligonucleotide with a sequence that extends beyond the 3' end of the immobilised primer, whose sequence is also the same sequence as the corresponding region at the end of the template. This extended portion can serve as a basis for extension of the immobilised primer, and thus the extended primer is complementary to the immobilised template strand. The extended primers may improve the efficiency of the strand resynthesis step due to their increased length. Once a complementary sequence of the first strand has been generated, the first strand can be removed from the surface.

Removal of the first template from the surface allows the newly single stranded second template to be sequenced, again from the 3' end. Thus, both ends of the original immobilised template can be sequenced. The first strand can be removed by a suitable orthogonal linearisation treatment step, such as diol cleavage or removal of an 8-oxo-G residue and after denaturation of the first template strand, a second sequencing primer can be hybridised to the second template strand, and the second template strand sequenced. This orthogonal linearisation strategy also allows reads from both ends of the template.

Selective removal, or linearisation of the first template strand can also be achieved in a number of other ways. The linearization to allow hybridization of a sequencing primer in solution does not have to leave a functional 3'-hydroxyl on the template strand, and can cleave either one strand or both strands. Thus, as used herein, the term "linearization" refers to the selective removal of a complementary strand. If one of the amplification primers is immobilised such that it can be cleaved from the surface, the resulting double stranded DNA can be made single stranded using heat or chemical denaturing conditions to give a single stranded molecule containing a primer hybridisation site. The single stranded molecule can be hybridised with a sequencing primer in solution to allow a sequencing read of the immobilised template strand. Said cleavage site is a site which allows controlled cleavage of the first template strand by chemical, enzymatic or photochemical means. Any suitable enzymatic, chemical or photochemical cleavage reaction may be used to cleave. A number of suitable methods are described in WO07010251,. The cleavage reaction may result in removal of a part or the whole of the strand being cleaved. Suitable cleavage means include, for example, restriction enzyme digestion, in which case the cleavage site is an appropriate restriction site for the enzyme which directs cleavage of one or both strands of a duplex template; RNase digestion or chemical cleavage of a bond between a deoxyribonucleotide and a ribonucleotide, in which case the cleavage site may include one or more ribonucleotides; chemical reduction of a disulphide linkage with a reducing agent (e.g. TCEP), in which case the cleavage site should include an appropriate disulphide linkage; chemical cleavage of a diol linkage with periodate, in which case the cleavage site should include a diol linkage; generation of an abasic site and subsequent hydrolysis, etc.

In one embodiment cleavage may occur at a cleavage site in one or both strands of a template polynucleotide duplex which comprises one or more or any combination of non-natural nucleotides, ribonucleotides or a non-nucleotide chemical modifications.

Suitable cleavage techniques for use in the method of the invention include, but are not limited to, the following, which are described in detail in WO2008041002: i) chemical cleavage, ii) cleavage of abasic sites, iii) cleavage of ribonucleotides, iv) photochemical cleavage, v) PCR stoppers, vi) cleavage of peptide linker, vii) enzymatic digestion.

A second sequencing primer is then hybridised to the copied strand of the template and a sequencing reaction proceeds via successive addition of nucleotides to the second sequencing primer as described above, resulting in determination of the sequence of a second region of the target polynucleotide.

The polynucleotide duplexes described herein form part of a single cluster or colony comprised of many such first and second duplexes, and the cluster or colony will itself typically form part of an array of many such clusters or colonies. The terms "cluster" and "colony" are used interchangeably throughout and refer to a discrete site on a solid support comprised of a plurality of identical immobilised nucleic acid strands and a plurality of identical immobilised complementary nucleic acid strands. The term "clustered array" refers to an array formed from such clusters or colonies. Each polynucleotide duplex on the array contains the same universal primer recognition regions to allow the same primers to be used to sequence every cluster. As explained above, a first sequencing primer is then hybridised to the first template strand and a sequencing reaction proceeds via successive incorporation of nucleotides or oligonucleotides to the first sequencing primer, resulting in determination of the sequence of a first region of the target polynucleotide.

A key feature is that both sequencing runs can occur in the same cluster or colony on a clustered array. On such an array each duplex within each colony will comprise the same double-stranded target polynucleotide, whereas different colonies may be formed of duplexes comprising different double-stranded target polynucleotides. In a particular embodiment at least 90%, more particularly at least 95% of the colonies on a given clustered array will be formed from template duplexes comprising different double-stranded target polynucleotides, although within each individual colony on the array all template duplexes will comprise the same double-stranded target polynucleotide.

The sequencing method outlined above are not limiting and essentially any sequencing methodology which relies on successive incorporation of nucleotides into a polynucleotide chain can be used. Suitable techniques include, for example, Pyrosequencing(TM), FISSEQ (fluorescent in situ sequencing), MPSS (massively parallel signature sequencing) and sequencing by ligation-based methods. The target double-stranded polynucleotide to be sequenced may be any polynucleotide that it is desired to sequence. The target polynucleotide may be of known, unknown or partially known sequence, such as, for example in re-sequencing applications. Using the template preparation method described in detail below it is possible to prepare arrays of templates starting from essentially any double-stranded target polynucleotide of known, unknown or partially known sequence. With the use of arrays it is possible to sequence multiple targets of the same or different sequence in parallel. A particular application of the pairwise method is in the sequencing of fragments of genomic DNA. The method provides particular advantages in the identification of genome rearrangements, since the two regions of sequence obtained for each target molecule using the method will be known to be linked within a certain distance of each other in the genome, depending on the size of the starting target molecule.

The present invention has identified that non-thermostable strand displacement polymerases can advantageously be used during the regeneration step after the first sequencing read and before the second sequencing read. In other words, a non-thermostable strand displacement polymerase can be used to resynthesise a complementary strand of a first template strand following sequencing of that strand. As explained above, this is called paired-end resynthesis.

"Non-thermostable polymerase" is intended to encompass polymerases that are optimised to operate at a lower temperature and contrast with thermostable polymerases like BST.

"Strand displacement polymerase" describes the ability to displace downstream DNA encountered during synthesis. The non-thermostable polymerases according to the present invention have an optimum incubation temperature below 55°C. In a further embodiment, non-thermostable polymerases according to the present invention have an optimum incubation temperature below 50°C, below 45°C, below 40°C, below 39°C, at or below 38°C, around 38°C or around 37°C. Particularly preferred non-thermostable polymerases have an optimum incubation temperature of around 38°C.

In a further embodiment, non-thermostable polymerases according to the present invention have an optimum activity temperature below 55°C. In a further embodiment, non-thermostable polymerases according to the present invention have an optimum activity temperature below 50°C, below 45°C, below 40°C, below 39°C, at or below 38°C, around 38°C or around 37°C. Particularly preferred non-thermostable polymerases have an optimum activity temperature of around 38°C.

Suitable non-thermostable strand displacement polymerases according to the present invention can be found, for example, through New England BioLabs, Inc. and include phi29, Bsu, Klenow, and DNA Polymerase I (E. coli) and functional fragments thereof. A particularly preferred polymerase is Bsu. In an alternative embodiment, the polymerase is the Bsu large fragment.

By using non-thermostable polymerases, the extension reaction can be carried out at a temperature of less than 55°C, preferably less than 50°C, preferably less than 40°C, preferably 38°C +/- 2°C, preferably 38°C +/- 1°C, preferably 38°C.

The use of strand displacement polymerases also leads to advantages and can overcome challenges with template strands re-annealing to themselves. The use of strand displacement polymerases assists in ensuring template strands anneal to the relevant primers to enable efficient extension.

The use of non-thermostable polymerases during the regeneration step after the first sequencing read leads to a number of advantages. By way of example, the use of thermostable BST has been previously used during this regeneration step requiring 12 regeneration cycles to achieve an optimum % resynthesis. In contrast, non-thermostable polymerases have been shown to achieve acceptable resynthesis after just three cycles. See, for example, the PETv2 3x5m protocol in the Examples which achieved 79.4% resynthesis after just 3 cycles.

The use of non-thermostable polymerases during paired end resynthesis is counterintuitive because the step is between two sequencing steps, both of which are carried out at high temperature using suitable SBS polymerases which may operate at 60 or 65 °C.

The use of non-thermostable polymerases during paired end resynthesis may also lead to further advantages if the same polymerases are used during initial cluster amplification to generate the cluster array which is subsequently sequenced. By way of example, if ExAmp (an amplification mix comprising non-thermostable strand displacement polymerase BSU) is used for both the initial cluster generation step and for the paired end resynthesis step, then the total number of reagents used within the machine is reduced and the cartridge complexity is also reduced. Such advantages can lead to COGS optimisation and protocol simplification.

In an embodiment, step (d) is repeated through multiple cycles of extension and denaturation. This may comprise 2, 3, 4, 5, 6, 7, 8, 9, 10, 11, 12, 13, 14, 15 or more cycles; preferably 2-5 cycles, 2-4 cycles, 3-5 cycles, 3 cycles or 4 cycles; most preferably 3 cycles. By way of contrast with a polymerase requiring higher numbers of cycles, the present invention can complete the regeneration step efficiently.

In an embodiment, each cycle may last for 1, 2, 3, 4, 5, 6, 7, 8, 9, 10, 11, 12, 13, 14, 15, 20, or 30 minutes. Particularly preferred cycle types are 5 minutes. In particular embodiments, step (d) may comprise the following cycles of extension and denaturation: 3x5mins; 3x30mins; 12x2mins; 6×4mins; 2x12mins or 3x5mins. In a particular embodiment, 3x5mins is preferred.

In a preferred embodiment, the extension reaction during pairwise sequencing comprises cycles of extension and denaturation.

Any suitable denaturation agent is encompassed by the present invention. Suitable denaturation agents include: acidic nucleic acid denaturants such as acetic acid, HCl, or nitric acid; basic nucleic acid denaturants such as NaOH; or other nucleic acid denaturants such as DMSO, formamide, betaine, guanidine, sodium salicylate, propylene glycol or urea. Preferred denaturation agents are formamide and NaOH, preferably formamide.

In an embodiment, denaturation is carried out substantially at the temperature of the extension reaction.

In a particularly preferred embodiment, the extension / denaturation cycle comprises three cycles of extension with two cycles of denaturation therebetween. In a further preferred embodiment, a denaturation cycle is carried out before the first extension cycle. Such an embodiment comprises three alternating cycles of denaturation and extension. Formamide is the most preferred denaturant.

In an embodiment, the extension reaction (d) is carried out at a lower temperature than the sequencing read steps. The sequencing steps will generally use a different polymerase (for example SBS polymerases) that are optimised to higher temperatures.

In an embodiment, the plurality of double stranded template polynucleotides are generated by an amplification reaction using the same polymerase as the polymerase used in the extension reaction step (d).

The plurality of double stranded template polynucleotides immobilised on said solid support in step (a) may preferably be a cluster. The present invention encompasses any method to generate said cluster, but one preferred methodology uses bridge amplification. In a preferred embodiment, said bridge amplification to generate the cluster uses the same non-thermostable strand displacement polymerase used for the step (d) extension reaction. Such an approach leads to further methodology streamlining since it can minimise the amplification mixtures used across the process as a whole.

Thus, a single non-thermostable strand displacement polymerase can be used for all amplification steps to generate or regenerate the immobilised template polynucleotides. A second polymerase can be used during the sequencing step which means that overall the entire process can be completed with two polymerases. Such a streamlined approach offers various process advantages, complexity reduction and COGS savings.

The terms "cluster" and "colony" are used interchangeably throughout and refer to a discrete site on a solid support comprised of a plurality of identical immobilised nucleic acid strands and a plurality of identical immobilised complementary nucleic acid strands. The term "clustered array" refers to an array formed from such clusters or colonies.

The present invention provides for a method for pairwise sequencing of first and second regions of a target double-stranded polynucleotide, wherein said first and second regions are in the same target double- stranded polynucleotide, said pairwise sequencing including the steps (a) to (f) as disclosed herein.

The method may be used for obtaining two linked or paired reads of sequencing information from each of the double-stranded template on a clustered array.

The present invention provides for a method of improving the data quality of a sequencing reaction, comprising carrying out an extension reaction as disclosed herein.

The present invention will now be described by way of the following non-limiting examples.

### EXAMPLES

Paired end sequencing can be carried out following the methodology described in WO 2008/041002 (see, for example, Example 13), with the following modifications described below following the first sequencing step of the target fragment. Following successful completion of the read 1 sequencing:
1. Deprotection of the flow cell surface bound i5 primers is carried out enzymatically using a resynthesis mix which comprises buffer and T4 Polynucleotide Kinase, such as JRM, which is available from Illumina, Inc.
2. The flow cell is next washed with ionic buffer, e.g. BB6 buffer, which is available from Illumina Inc.
3. Clusters are denatured with Low-bias Denaturation Reagent (LDR -100% formamide) to remove the extended sequencing primer from read 1.
4. Flow cell temperature is brought to 38°C.
5. In order to regenerate double stranded clusters (Resynthesis), three subsequent rounds of LDR (100% formamide) and ExAmp (amplification mix comprising non-thermostable strand displacement polymerase BSU) are cycled isothermally at 38°C as follows. LDR and ExAmp are available from Illumina, Inc.
   5a. LDR is flushed through the flow cell with a large flush factor intended to fully displace the contents of the flow cell.
   5b. ExAmp is next flushed across the flow cell maintaining a large flush factor and incubated for 5 minutes.
   5c. Steps 5a and 5b are repeated for a total of three rounds of amplification.
6. The newly double stranded clusters are next linearized for Read 2 and primer hybridization is carried out as in WO 2008/041002.

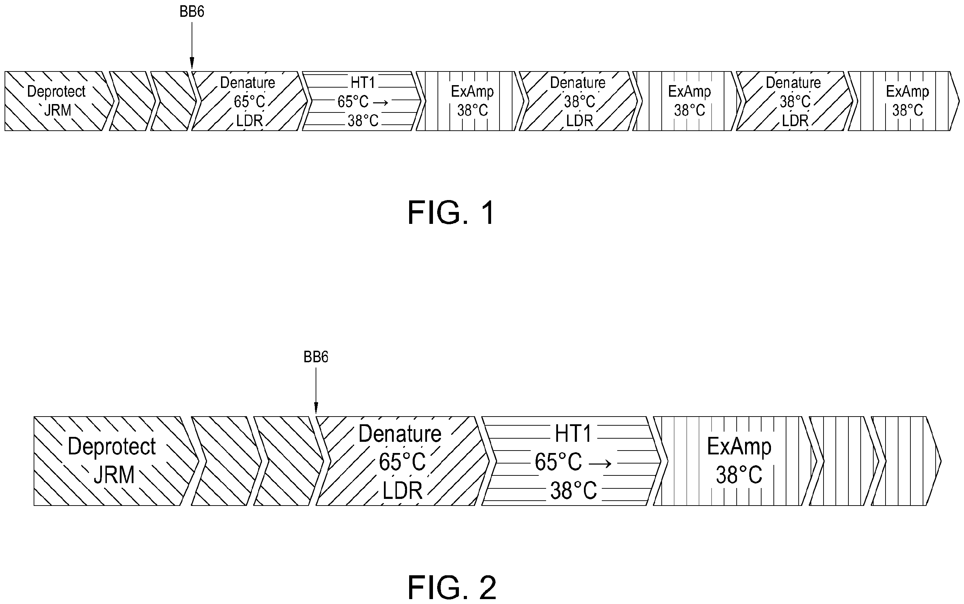

A graphical representation of a workflow according to the present invention is shown in FIG. 1. The workflow PETv2 shows deprotection with JRM followed by a buffer wash. The cluster is then denatured with LDR followed by a thermal cycle down to 38°C. Thereafter follows three rounds of regeneration and denaturation using ExAmp (containing BSU) and LDR (100% formamide).

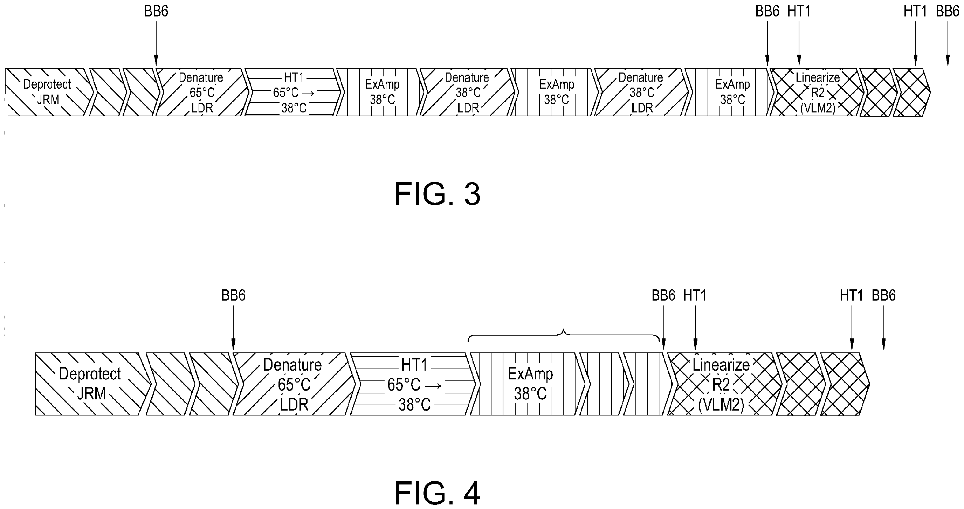

An alternative workflow PETv1 is shown in FIG. 2 which is similar to the FIG. 1 workflow but which does not include denaturation with LDR between regeneration and instead proceeds with x3 consecutive rounds of regeneration. FIGs. 3 and 4 mirror FIGs. 1 and 2 but further include linearization.

In embodiments, SSC may also be used in protocols according to the present invention. For example, clusters may be washed before and/or after resynthesis with SSC.

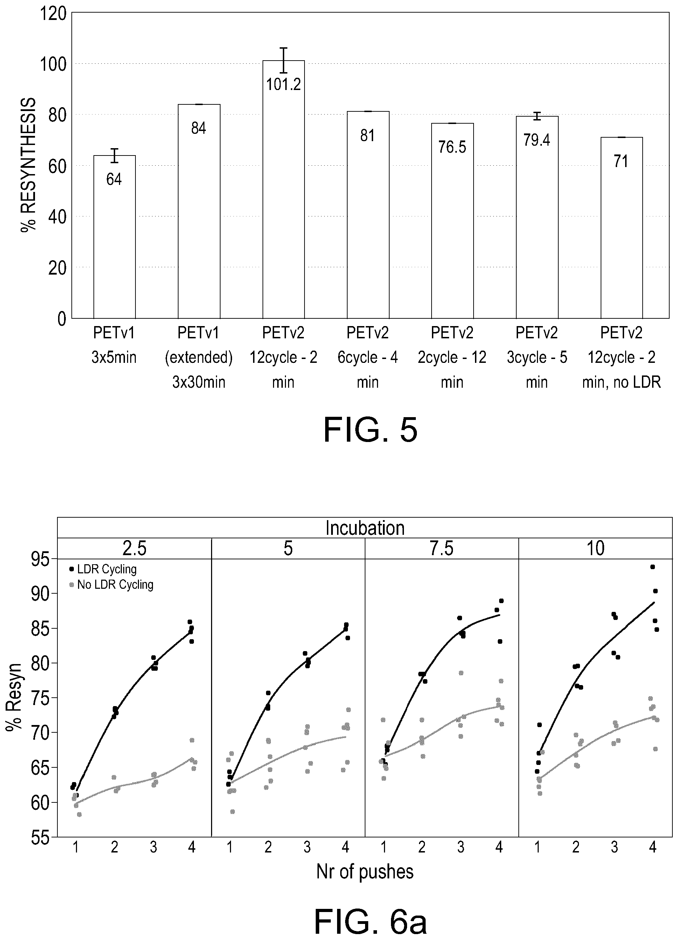

The regeneration efficiency of these workflows was evaluated against other potential workflows and the results are shown in FIG. 5 which show % resynthesis calculated from HSX sequencing runs with paired end turn protocols according to the present invention as described in Table 1 below.

**Table 1**

| **Details** | **%Resyn** | **SD** | **n** |
|---|---|---|---|
| PETv1 3x5min | **64** | 2.6 | (n=15) |
| PETv1 (extended) 3x30min | **84** | NA | (n=1) |
| PETv2 12cycle - 2 min | **101.2** | 4.9 | (n=3) |
| PETv2 6cycle - 4 min | **81** | NA | (n=1) |
| PETv2 2cycle - 12 min | **76.5** | NA | (n=2) |
| PETv2 3cycle - 5 min | **79.4** | 1.3 | (n=3) |
| PETv2 12cycle - 2 min, no LDR | **71** | NA | (n=1) |

The initial protocol tested was PETv1 following a 3x5min cycle. This achieved 64% resynthesis which is considered acceptable but is not optimised. Increasing the incubation time to 3x30min improved % resynthesis to 84%, and increasing both the incubation time and the number of chemistry cycles also led to a positive impact on % resynthesis ('PETv2 12 cycles - 2min, no LDR' improved % resynthesis to 71%).

The use of consecutive regeneration/denaturing cycles, in the example with LDR, further improved % resynthesis to 76.5 to 101.2% depending on conditions.

It can be seen that increasing ExAmp incubation time, number of chemistry cycles and/or frequency of LDR use leads to a positive impact on % resynthesis. Typically, the selected protocol will achieve -80% resynthesis or more, but the preferential protocol will be a trade of % resynthesis vs time vs protocol complexity and reagent use.

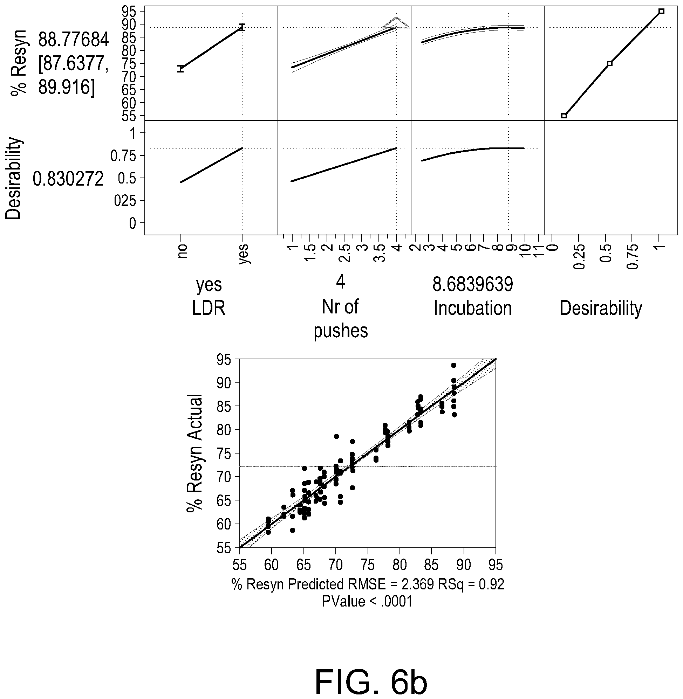

The protocol optimisation was also considered using iCbot based DOE and the results are shown in FIGs. 6A and 6B.

FIG. 6A shows that: i) including LDR cycling has a significant effect on % resynthesis; i) increasing the number of cycling steps also improves % resynthesis. This is particularly seen when LDR cycling is used; iii) increasing the incubation time has a positive but less pronounced effect on % resynthesis, suggesting that shorter run times are acceptable.

FIG. 6B shows a model fit of the experiment identifying the optimised parameters which are: LDR = yes; 4 pushes; and an incubation time of 8.7 minutes. As discussed herein, the final optimised protocol will not only take into account % resynthesis but also overall optimisation in terms of speed, reagent use and efficiency.

In conclusion, the present invention has identified that the extension reaction during pairwise sequencing to regenerate the template during sequencing runs can be carried out using a non-thermostable strand displacement polymerase at a temperature of less than 55 °C. This leads to process advantages. Additionally, if the same polymerase is used for both the extension reaction and also the initial cluster generation then the overall process can be simplified and the number of reagents and protocol steps be reduced.

## Claims

1. A method for carrying out a strand resynthesis extension reaction during pairwise sequencing, wherein astrand resynthesis extension reaction is carried out between a first sequencing read and a second sequencing read, and wherein said strand resynthesis extension reaction extends one or more immobilised primers to copy a first template strand to generate a second immobilised template strand; **characterised in that** the strand resynthesis extension reaction is carried out using a non-thermostable strand displacement polymerase at a temperature of less than 55 °C, wherein said first template strands are a cluster, and wherein the same non-thermostable strand displacement polymerase is used during initial cluster generation and the strand resynthesis extension reaction.

2. The method of claim 1, wherein the non-thermostable polymerase has an optimum incubation temperature and/or optimum activity temperature below 55 °C, preferably below 50°C, below 45°C, below 40°C, below 39°C, at or below 38°C, around 38°C or around 37°C; particularly preferably around 38°C.

3. The method of any preceding claim, wherein the extension reaction is carried out at a temperature of less than 55 °C, preferably less than 50°C, preferably less than 40°C, preferably 38°C +/- 2°C, preferably 38°C +/- 1°C, preferably 38°C.

4. The method of any preceding claim, wherein the non-thermostable polymerase is Bsu, phi29, Klenow, or DNA Polymerase I (E. coli), preferably Bsu or a functional fragment thereof.

5. The method of any preceding claim, wherein the strand resynthesis extension reaction is repeated through multiple cycles of extension and denaturation, wherein preferably, the method comprises 2, 3, 4, 5, 6, 7, 8, 9, 10, 11, 12, 13, 14, 15 or more cycles; preferably 2-5 cycles, 2-4 cycles, 3-5 cycles, 3 cycles or 4 cycles; most preferably 3 cycles.

6. The method of any preceding claim, wherein each cycle may last for 1, 2, 3, 4, 5, 6, 7, 8, 9, 10, 11, 12, 13, 14, 15, 20, or 30 minutes, wherein preferably the method comprises the following cycles of extension and denaturation: 3x5mins; 3x30mins; 12x2mins; 6x4mins; 2x12mins or 3x5mins; preferably 12x2mins.

7. The method of any preceding claim, wherein denaturation is carried out substantially at the temperature of the extension reaction, wherein preferably denaturation is carried out using acidic nucleic acid denaturants such as acetic acid, HCl, or nitric acid; basic nucleic acid denaturants such as NaOH; or other nucleic acid denaturants such as DMSO, formamide, betaine, guanidine, sodium salicylate, propylene glycol or urea; preferably formamide and NaOH, particularly preferably formamide.

8. The method according to any preceding claim, wherein the strand resynthesis extension reaction is carried out at a lower temperature than the sequencing read steps.

9. The method according to any preceding claim, wherein said cluster was initially generated by bridge amplification.

10. The method according to any preceding claim, wherein at least one of the immobilised primers is blocked at the 3' end, and the block is removed prior to the strand resynthesis extension reaction, wherein preferably the block is a phosphate group and is treated with a phosphatase to remove the block.

11. The method according to any preceding claim, further comprising a step of treating with a restriction enzyme prior to the strand resynthesis extension reaction to shorten the immobilised primer and release a free 3' hydroxyl for extension.

12. The method according to any preceding claim, wherein the immobilised primer is extended prior to the strand resynthesis extension reaction, wherein preferably the immobilised primer is extended by hybridisation of a non-immobilised complementary sequence with a 5'-overhang, and the immobilised primer is extended to copy the overhang.

13. A method for pairwise sequencing of first and second regions of a target double-stranded polynucleotide, wherein said first and second regions are in the same target double-stranded polynucleotide, said pairwise sequencing including the steps as disclosed in any one of claims 1 to 12.

14. Use of the method of claim 13 for obtaining two linked or paired reads of sequencing information from each of the double-stranded template on a clustered array.

15. A method of improving the data quality of a sequencing reaction, comprising carrying out an extension reaction as claimed in any one of claims 1 to 12.

## Patentansprüche

1. Verfahren zum Ausführen einer Strangresynthese-Verlängerungsreaktion während paarweisen Sequenzierens, wobei eine Strangresynthese-Verlängerungsreaktion zwischen einem ersten Sequenzierungs-Read und einem zweiten Sequenzierungs-Read ausgeführt wird und wobei die Strangresynthese-Verlängerungsreaktion einen oder mehrere immobilisierte Primer verlängert, um einen ersten Matrizenstrang zu kopieren, um einen zweiten immobilisierten Matrizenstrang zu erzeugen; **dadurch gekennzeichnet, dass** die Strangresynthese-Verlängerungsreaktion unter Verwendung einer nichtthermostabilen Strangverdrängungspolymerase bei einer Temperatur von weniger als 55 °C ausgeführt wird, wobei die ersten Matrizenstränge ein Cluster sind und wobei dieselbe nichtthermostabile Strangverdrängungspolymerase während der anfänglichen Clustererzeugung und der Strangresynthese-Verlängerungsreaktion verwendet wird .

2. Verfahren nach Anspruch 1, wobei die nichtthermostabile Polymerase eine optimale Inkubationstemperatur und/oder optimale Aktivitätstemperatur unter 55 °C, bevorzugt unter 50 °C, unter 45 °C, unter 40 °C, unter 39 °C, bei oder unter 38 °C, etwa 38 °C oder etwa 37 °C, besonders bevorzugt etwa 38 °C aufweist.

3. Verfahren nach einem der vorhergehenden Ansprüche, wobei die Verlängerungsreaktion bei einer Temperatur von weniger als 55 °C, bevorzugt weniger als 50 °C, bevorzugt weniger als 40 °C, bevorzugt 38 °C +/- 2 °C, bevorzugt 38 °C +/- 1 °C, bevorzugt 38 °C ausgeführt wird.

4. Verfahren nach einem der vorhergehenden Ansprüche, wobei die nichtthermostabile Polymerase Bsu, phi29, Klenow oder DNA-Polymerase I (E. coli), bevorzugt Bsu oder ein funktionelles Fragment davon ist.

5. Verfahren nach einem der vorhergehenden Ansprüche, wobei die Strangresynthese-Verlängerungsreaktion durch mehrere Verlängerungs- und Denaturierungszyklen hindurch wiederholt wird, wobei das Verfahren bevorzugt 2, 3, 4, 5, 6, 7, 8, 9, 10, 11, 12, 13, 14, 15 oder mehr Zyklen, bevorzugt 2-5 Zyklen, 2-4 Zyklen, 3-5 Zyklen, 3 Zyklen oder 4 Zyklen, am bevorzugtesten 3 Zyklen umfasst.

6. Verfahren nach einem der vorhergehenden Ansprüche, wobei jeder Zyklus 1, 2, 3, 4, 5, 6, 7, 8, 9, 10, 11, 12, 13, 14, 14, 15, 20 oder 30 Minuten andauern kann, wobei das Verfahren bevorzugt die folgenden Verlängerungs- und Denaturierungszyklen: 3x5 min, 3x30 min, 12x2 min, 6x4 min, 2x12 min oder 3x5 min, bevorzugt 12x2 min umfasst.

7. Verfahren nach einem der vorhergehenden Ansprüche, wobei die Denaturierung im Wesentlichen bei der Temperatur der Verlängerungsreaktion ausgeführt wird, wobei die Denaturierung bevorzugt unter Anwendung saurer Nukleinsäure-Denaturierungsmittel wie beispielsweise Essigsäure, HCl oder Salpetersäure, basischer Nukleinsäuredenaturierungsmittel wie beispielsweise NaOH oder anderer Nukleinsäuredenaturierungnittel wie beispielsweise DMSO, Formamid, Betain, Guanidin, Natriumsalicylat, Propylenglykol oder Harnstoff, bevorzugt Formamid und NaOH, besonders bevorzugt Formamid ausgeführt wird.

8. Verfahren nach einem der vorhergehenden Ansprüche, wobei die Strangresynthese-Verlängerungsreaktion bei einer niedrigeren Temperatur als die Sequenzierungs-Read-Schritte ausgeführt wird.

9. Verfahren nach einem der vorhergehenden Ansprüche, wobei der Cluster anfänglich durch Brückenamplifikation erzeugt worden ist.

10. Verfahren nach einem der vorhergehenden Ansprüche, wobei mindestens einer der immobilisierten Primer am 3'-Ende blockiert ist und der Block vor der Strangresynthese-Verlängerungsreaktion entfernt wird, wobei der Block bevorzugt eine Phosphatgruppe ist und mit einer Phosphatase zum Entfernen des Blocks behandelt wird.

11. Verfahren nach einem der vorhergehenden Ansprüche, ferner einen Schritt des Behandelns mit einem Restriktionsenzym vor der Strangresynthese-Verlängerungsreaktion umfassend, um den immobilisierten Primer zu verkürzen und ein freies 3'-Hydroxyl zur Verlängerung freizusetzen.

12. Verfahren nach einem der vorhergehenden Ansprüche, wobei der immobilisierte Primer vor der Strangresynthese-Verlängerungsreaktion verlängert wird, wobei der immobilisierte Primer bevorzugt durch Hybridisierung einer nichtimmobilisierten komplementären Sequenz mit einem 5'-Überhang verlängert wird und der immobilisierte Primer verlängert wird, um den Überhang zu kopieren.

13. Verfahren zum paarweisen Sequenzieren erster und zweiter Regionen eines doppelsträngigen Targetpolynukleotids, wobei die ersten und zweiten Regionen sich in demselben doppelsträngigen Targetpolynukleotid befinden, wobei das paarweise Sequenzieren die Schritte wie in einem der Ansprüche 1 bis 12 offenbart, einschließt.

14. Verwendung des Verfahrens nach Anspruch 13 zum Erhalten von zwei verknüpften oder gepaarten Reads von Sequenzierinformationen von jeder der doppelsträngigen Matrizen an einer geclusterten Anordnung.

15. Verfahren zum Verbessern der Datenqualität einer Sequenzierreaktion, umfassend das Ausführen einer Verlängerungsreaktion nach einem der Ansprüche 1 bis 12.

## Revendications

1. Procédé permettant la réalisation d'une réaction d'extension par resynthèse de brin pendant un séquençage par paires, dans lequel une réaction d'extension par resynthèse de brin est réalisée entre une première lecture de séquençage et une deuxième lecture de séquençage et dans lequel ladite réaction d'extension par resynthèse de brin étend une ou plusieurs amorces immobilisées pour copier un premier brin de matrice pour générer un deuxième brin de matrice immobilisé; **caractérisé en ce que** la réaction d'extension par resynthèse de brin est réalisée en utilisant une polymérase de déplacement de brin non thermostable à une température de moins de 55 °C, dans lequel lesdits premiers brins de matrice sont un groupe et dans lequel la même polymérase de déplacement de brin non thermostable est utilisée pendant la génération initiale de groupe et la réaction d'extension par resynthèse de brin.

2. Procédé selon la revendication 1, dans lequel la polymérase non thermostable a une température d'incubation optimale et/ou une température d'activité optimale en dessous de 55 °C, de préférence en dessous de 50 °C, en dessous de 45 °C, en dessous de 40 °C, en dessous de 39 °C, à ou en dessous de 38 °C, autour de 38 °C ou autour de 37 °C, de manière particulièrement préférée autour de 38 °C.

3. Procédé selon l'une quelconque des revendications précédentes, dans lequel la réaction d'extension est réalisée à une température de moins de 55 °C, de préférence moins de 50 °C, de préférence moins de 40 °C, de préférence 38 °C +/- 2 °C, de préférence 38 °C +/- 1 °C, de préférence 38 °C.

4. Procédé selon l'une quelconque des revendications précédentes, dans lequel la polymérase non thermostable est Bsu, phi29, Klenow ou une ADN polymérase I (E. coli), de préférence Bsu ou un fragment fonctionnel de celle-ci.

5. Procédé selon l'une quelconque des revendications précédentes, dans lequel la réaction d'extension par resynthèse de brin est répétée par plusieurs cycles d'extension et de dénaturation, où, de préférence, le procédé comprend 2, 3, 4, 5, 6, 7, 8, 9, 10, 11, 12, 13, 14, 15 cycles ou plus; de préférence 2-5 cycles, 2-4 cycles, 3-5 cycles, 3 cycles ou 4 cycles, le plus préférablement 3 cycles.

6. Procédé selon l'une quelconque des revendications précédentes, dans lequel chaque cycle peut durer 1, 2, 3, 4, 5, 6, 7, 8, 9, 10, 11, 12, 13, 14, 15, 20 ou 30 minutes, où, de préférence, le procédé comprend les cycles d'extension et de dénaturation suivants: 3x5 mins; 3x30 mins; 12x2 mins; 6x4 mins; 2x12 mins ou 3x5 mins; de préférence 12x2 mins.

7. Procédé selon l'une quelconque des revendications précédentes, dans lequel la dénaturation est réalisée substantiellement à la température de la réaction d'extension, de préférence dans lequel la dénaturation est réalisée en utilisant des dénaturants d'acide nucléique acides tels que l'acide acétique, HCl ou l'acide nitrique; des dénaturants d'acide nucléique basiques tels que NaOH; ou d'autres dénaturants d'acide nucléique tels que le DMSO, le formamide, la bétaïne, la guanidine, la salicylate de sodium, le propylène glycol ou l'urée; de préférence le formamide et le NaOH, de manière particulièrement préférée le formamide.

8. Procédé selon l'une quelconque des revendications précédentes, dans lequel la réaction d'extension par resynthèse de brin est réalisée à une température inférieure à celle des étapes de lecture de séquençage.

9. Procédé selon l'une quelconque des revendications précédentes, dans lequel ledit groupe a été généré initialement par une amplification de pont.

10. Procédé selon l'une quelconque des revendications précédentes, dans lequel au moins une des amorces immobilisées est bloquée à l'extrémité 3' et le bloc est retiré avant la réaction d'extension par resynthèse de brin, de préférence dans lequel le bloc est un groupe phosphate et il est traité avec une phosphatase pour retirer le bloc.

11. Procédé selon l'une quelconque des revendications précédentes, comprenant en outre une étape de traitement avec une enzyme de restriction avant la réaction d'extension par resynthèse de brin pour raccourcir l'amorce immobilisée et lâcher un hydroxyle 3' libre pour une extension.

12. Procédé selon l'une quelconque des revendications précédentes, dans lequel l'amorce immobilisée est allongée avant la réaction d'extension par resynthèse de brin, de préférence dans lequel l'amorce immobilisée est allongée par l'hybridation d'une séquence complémentaire non immobilisée avec un surplomb en 5' et l'amorce immobilisée est allongée pour copier le surplomb.

13. Procédé pour le séquençage par paires de première et deuxième régions d'un polynucléotide cible à double brin, dans lequel lesdites première et deuxième régions sont dans le même polynucléotide cible à double brin, ledit séquençage par paires incluant les étapes telles que divulguées dans l'une quelconque des revendications 1 à 12.

14. Utilisation du procédé selon la revendication 13 pour l'obtention de deux lectures liées ou appariées d'information de séquençage à partir de chacune des matrices à double brin sur un arrangement en groupe.

15. Procédé pour l'amélioration de la qualité des données d'une réaction de séquençage, comprenant la réalisation d'une réaction d'extension selon l'une quelconque des revendications 1 à 12.
